# EUROPEAN PATENT SPECIFICATION

(11) **EP 3 967 766 B1**
(45) Date of publication and mention of the grant of the patent: **10.04.2024**
(21) Application number: 19927900.1
(22) Date of filing: 24.10.2019
(51) Int. Cl.: C12Q 1/6895, C12Q 1/686

(54) **PRIMER PROBE COMBINATION, KIT, DETECTION METHOD AND APPLICATION FOR CANDIDA SPECIES DETECTION**
PRIMERSONDENKOMBINATION, KIT, NACHWEISVERFAHREN UND ANWENDUNG FÜR DIE DETEKTION VON CANDIDA-SPEZIES
COMBINAISON DE SONDES D'AMORCE, KIT, PROCÉDÉ DE DÉTECTION ET APPLICATION POUR LA DÉTECTION D'ESPÈCES DE CANDIDA

(30) Priority: 07.05.2019 CN 201910375345
(43) Date of publication of application: 16.03.2022
(73) Proprietor: Dynamiker Biotechnology (Tianjin) Co., Ltd., Tianjin 300467 (CN)
(72) Inventor: WANG, Zhixian, Tianjin 300467 (CN); YAN, Xiangyan, Tianjin 300467 (CN); SHENG, Changzhong, Tianjin 300467 (CN); ZHOU, Zeqi, Tianjin 300467 (CN); SU, Yan, Tianjin 300467 (CN)
(74) Representative: JD&P Patent Attorneys Joanna Dargiewicz & Partners
(86) International application number: PCT/CN2019/112917
(87) International publication number: WO 2020/224194

(56) References cited:
- WO-A2-2009/150241
- CN-A- 109 055 502
- CN-A- 109 487 000
- CN-A- 109 666 755
- CN-A- 109 971 883
- US-A1- 2013 280 708
- KHAN ZIAUDDIN ET AL: "Real-time LightCycler polymerase chain reaction and melting temperature analysis for identification of clinically important Candida spp", JOURNAL OF MICROBIOLOGY, IMMUNOLOGY, AND INFECTION = WEI MIAN YU GAN RAN ZA ZHI,, vol. 42, no. 4, 1 August 2009 (2009-08-01) , pages 290-295, XP009151748,
- HSU MIN-CHIH ET AL: "Species identification of medically important fungi by use of real-time LightCycler PCR", JOURNAL OF MEDICAL MICROBIOLOGY, CHURCHILL LIVINGSTONE [ETC.], vol. 52, no. Pt 12, 1 December 2003 (2003-12-01), pages 1071-1076, XP002569841, ISSN: 0022-2615, DOI: 10.1099/JMM.0.05302-0

## Description

### TECHNICAL FIELD

The present invention belongs to the technical field of biology, relates to a primer probe combination, a kit, a detection method and an application for detecting separate *Candida* types, and particularly relates to a primer probe combination, a kit, a detection method and an application for separately detecting *Candida albicans, Candida tropicalis, Candida parapsilosis, Candida krusei* and *Candida glabrata.*

### BACKGROUND OF THE PRESENT INVENTION

*Candida* is one of the most common conditioned pathogens in fungi, and mainly includes *Candida albicans, Candida tropicalis, Candida glabrata, Candida parapsilosis* and *Candida krusei.* The *Candida* accounts for over 80% of clinical medical samples. The *Candida* is often parasitic on human skin, oral cavity, vagina and intestinal mucosa. Candidiasis may be easily caused when immune functions of the body are reduced. Since a time period of distinguishing different *Candida* types by a culture method is long, *Candida* infection cannot be rapidly treated with clinically common broad-spectrum antifungal agents. Different *Candida* types have different antibiotic resistances. Different influences may be brought to the body if a unified treatment plan is adopted without distinguishing the types. Different strains are often detected by a polymerase chain reaction (PCR) in the prior art.

CN109055502A discloses a rapid multiple PCR identification and diagnosis detection method for invasive fungal infections based on cell free DNA (cfDNA). The method can identify invasive infections caused by clinically common and high-incidence *Candida albicans, Candida tropicalis, Candida parapsilosis, Candida krusei, Candida glabrata* and *Aspergillus fumigtus.* In the present invention, amplification primers are designed according to feature genome regions of various fungal genera and types; fluorescence detection probes that can distinguish the *Candida* types are designed according to amplified fragments; to-be-detected samples are subjected to real time PCR; and advantages such as high sensitivity of nested PCR and high specificity and multi-target property of multiple fluorescent hybridization probe PCR are integrated for identifying fungal strains. Although multiple pathogenic bacteria are identified by a one-step single-tube reaction in the method, multiple primers and probes exist in the reaction system, and analysis is performed by virtue of different fluorescence channels, thereby increasing detection cost and cycles.

CN101509037A discloses a rapid diagnostic kit for detecting four/more than four kinds of invasive *Candida* and an application method of the kit. The kit includes specific primers, a fluorescent quantitative PCR reaction solution of fluorescence probes corresponding to at least four kinds of *Candida,* and positive quality control and negative quality control of the at least four kinds of *Candida*; and the four kinds of *Candida* are judged according to Ct values detected by a fluorescent quantitative PCR detector. By virtue of dual control of high-specificity amplification of the primers and high specific hybridization of fluorescence probes, a false-positive rate is low. However, different fluorescence probes have different fluorophore modifications and detection channels, and thus detection cost and time are increased.

CN108034745A discloses a primer probe combination for detecting four kinds of *Candida,* and belongs to the technical field of microbiological detection. The primer probe combination includes a pair of primers and a probe. The pair of primers includes a forward primer and a reverse primer; and the probe is a sequence labeled with reporter fluorophores and quencher fluorophores. The pair of primers and the probe provided in the present invention can specifically and simultaneously detect four kinds of *Candida* such as *Candida albicans, Candida tropicalis, Candida glabrata* and *Candida parapsilosis.* However, specific strains corresponding to positive samples cannot be distinguished in the invention, i.e., types of the *Candida* cannot be distinguished.

US 2013/280708 A1 features compositions and methods for the detection of *Candida* species in biological samples, such as human tissue samples. The invention features species specific nucleic acid probes that can hybridize to target nucleic acid molecules in *Candida* species and can be used in a variety of detection assays, such as fluorescence based assays or NMR based assays. The compositions and methods of the invention can be used to rapidly and accurately detect one or more *Candida* species in patients and can be used to assist in point-of care-decisions for treating *Candida* infections.

WO 2009/150241 A2 relates to a diagnostic kit for a yeast or fungal species comprising at least one oligonucleotide probe capable of binding to at least a portion of the eIF2y gene or its corresponding mRNA.

KHAN ZIAUDDIN ET AL, "Real-time LightCycler polymerase chain reaction and melting temperature analysis for identification of clinically important Candida spp", J. OF MICROBIOLOGY, IMMUNOLOGY, AND INFECTION = WEIMIAN YU GAN RAN ZA ZHI, Vol. 42, No. 4, (2009-08-01), pages 290-295, XP009151748 discloses to use the LightCycler PCR System. The targets of genomic DNA isolated from the reference strains of 6 *Candida spp.* were amplified using genus- and species-specific primers, and detected in real-time employing SYBR Green fluorescent dye. The identity of *Candida spp.* was established by melting curve analysis. A similar analysis was performed with clinical isolates (n = 72) previously identified by conventional methods.

Therefore, providing a method that is excellent in specificity, efficient, rapid, and high in sensitivity and can distinguish different *Candida* types has significances, and lays the foundation for accurate medication and epidemic disease study.

### SUMMARY OF PRESENT INVENTION

With respect to defects and actual requirements in the prior art, the present invention provides a primer probe combination, a kit, a detection method and an application for detecting separate *Candida* types. Whether infection is caused by *Candida* can be detected and determined at a time; and *Candida albicans*, *Candida tropicalis*, *Candida parapsilosis*, *Candida krusei* and *Candida glabrata* can be simultaneously distinguished in the same fluorescence channel.

To achieve the purpose, technical solutions of the present invention are as follows:

In a first aspect, the present invention provides a primer probe combination for separately detecting *Candida.* The primer probe combination includes specific primers, detection probes and complementary probes specified at *Candida albicans, Candida tropicalis, Candida parapsilosis, Candida krusei* and *Candida glabrata* respectively, wherein the complementary probes and the detection probes are in incomplete complementary pairing.

Nucleotide sequences of the specific primers of the *Candida albicans* are shown as SEQ ID NO.1-2; nucleotide sequences of the specific primers of the *Candida tropicalis* are shown as SEQ ID NO.3-4; nucleotide sequences of the specific primers of the *Candida parapsilosis* are shown as SEQ ID NO.5-6; nucleotide sequences of the specific primers of the *Candida krusei* are shown as SEQ ID NO.7-8; and nucleotide sequences of the specific primers of the *Candida glabrata* are shown as SEQ ID NO.9-10.

A nucleotide sequence of the detection probe of the *Candida albicans* is shown as SEQ ID NO. 11; a nucleotide sequence of the detection probe of the *Candida tropicalis* is shown as SEQ ID NO. 12; a nucleotide sequence of the detection probe of the *Candida parapsilosis* is shown as SEQ ID NO. 13; a nucleotide sequence of the detection probe of the *Candida krusei* is shown as SEQ ID NO. 14; and a nucleotide sequence of the detection probe of the *Candida glabrata* is shown as SEQ ID NO.15.

A nucleotide sequence of the complementary probe of the *Candida albicans* is shown as SEQ ID NO. 16; a nucleotide sequence of the complementary probe of the *Candida tropicalis* is shown as SEQ ID NO. 17; a nucleotide sequence of the complementary probe of the *Candida parapsilosis* is shown as SEQ ID NO. 18; a nucleotide sequence of the complementary probe of the *Candida krusei* is shown as SEQ ID NO. 19; and a nucleotide sequence of the complementary probe of the *Candida glabrata* is shown as SEQ ID NO.20.

A quencher is modified at the 3' end of the detection probe of the *Candida albicans, Candida tropicalis, Candida parapsilosis, Candida krusei* and *Candida glabrata*; and a fluorophore is modified at the 5' end of the detection probe of the *Candida albicans, Candida tropicalis, Candida parapsilosis, Candida krusei* and *Candida glabrata*; the fluorophores modified at the 5' ends of the detection probes are the same.

In the present invention, specific target sequences of common *Candida albicans, Candida tropicalis, Candida parapsilosis, Candida krusei* and *Candida glabrata* in invasive *Candida* are simultaneously amplified by multiple PCR, and analyzed and identified by a specially designed Taqman probe melting curve, thereby realizing multiple detections of the five kinds of common invasive *Candida.* The primer probe combination in the present invention is excellent in specificity and high in sensitivity and realizes separate detection of strains of the *Candida.*

In the present invention, the detection probes and the complementary probes are in incomplete complementary pairing. Non-complementary bases are not located in 5 base sequences at 5' or 3' ends of the primers, and non-complementary locations cannot be continuous. Tm values of the detection probe and the complementary probe in incomplete complementary pairing are calculated according to a Tm value calculation formula of the nearest neighbor method. The formula is as follows: Tm=ΔH*/(ΔS*+RlnC_{T}). In the formula, ΔH* and ΔS* are respectively standard enthalpy change and entropy change of a hybridization reaction; R is a gas constant of 1.987 cal /kmol; and C_{T} is a molar concentration of DNA molecules (when the DNA molecules are asymmetric sequences, the molar concentration is CT/4). A probe of the designed Tm values of the detection probe and the complementary probe is designed by utilizing the formula, and a series of complementary probe and detection probe combinations that can be clearly distinguished by the Tm values are further designed, thereby distinguishing different kinds of *Candida* by different Tm values in the same fluorescence channel.

Multiple pairs of primers and probes exist in the same reaction system; for detecting different target sequences, stability, reaction conditions and amplification efficiency among various sequences need to be balanced; with the increasing number of detected bacteria seeds, design difficulty of the primer probe combination is significantly increased, and the design is restricted by multiple conditions. In the present invention, by virtue of multiple sequence alignment specified at the *Candida albicans, Candida tropicalis, Candida parapsilosis, Candida krusei* and *Candida glabrata,* the specific primers are designed, so that the multiple primers can stably exist in the same reaction system, and the amplification efficiency is consistent, thereby increasing the detection specificity and sensitivity and avoiding cross impact among the primers.

In the present invention, by aligning lots of nucleotide sequences on NCBI by virtue of ClustaX, the scope of aligned strains is as follows: 430 sequences of *Candida albicans* LC016565.1, HE860439.1, HE860438.1, LC201976.1, HG916992.1 and the like; 800 sequences of *Candida parapsilosis* AB109223.1, LT596112.1, LN864561.1, FM172980.1, LC390002.1 and the like; 350 sequences of *Candida tropicalis* KX664485.1, LT837798.1, JN542555.1, HM222942.1, FJ515173.1 and the like; 177 sequences of *Candida krusei* KU987874.1, KP674518.1, KX912139.1, FJ515204.1, EF568018.1 and the like; and 291 sequences of *Candida glabrata* HE993756.1, KU936094.1, KP780450.1, KM603625.1, LC011411.1, HG970737.1 and the like. By virtue of alignment of the 2048 sequences, highly conserved regions in the *Candida* types can be obtained to the full extent, thereby obtaining highly conserved regions and variable regions of different clinical isolates, further guiding primer design locations and increasing sensitivity of different isolates of different bacteria seeds. Selection of the target sequences is of great importance on the design of the primer probe combination. Consistent stability and amplification efficiency among different primers and probes and detection specificity and sensitivity need to be met simultaneously, and consistency of the reaction conditions should be further met. The more the types of the detected *Candida* are, the more the factors needing to be considered are. Meanwhile, the designed primer probe combination specified at detection of the five kinds of *Candida* is not equivalent to a primer probe combination specified at a single strain.

In the present invention, sequence regions such as 18S rRNA and 28S rRNA are preferred in regions of the specific target sequences of five kinds of to-be-detected *Candida.* Five Taqman probes and five complementary sequences thereof are respectively designed and prepared; and a pair of primer sequences is respectively designed at the periphery of the detection probe sequences, and the pair of primers includes a forward primer and a reverse primer. Specific targets are subjected to single or multiple single-color or multiple multicolor real-time fluorescent quantitative PCR amplification reactions with the designed primers.

In the present invention, a complementary probe is respectively designed specified at the detection probe of the five different kinds of *Candida.* The complementary probes and the detection probes are in incomplete complementary pairing and can form a double-stranded probe, so that the double-stranded probe specified at the different kinds of *Candida* has different melting properties; and the five different kinds of *Candida* can be distinguished by the characteristic melting temperature (Tm).

Preferably, the primer probe combination further includes an internal reference system.

Preferably, the internal reference system includes internal reference primers and an internal reference probe.

Preferably, nucleotide sequences of the internal reference primers are shown as SEQ ID NO.21-22.

Preferably, a nucleotide sequence of the internal reference probe is shown as SEQ ID NO.23.

Preferably, the fluorophore includes any one or a combination of at least two of ALEX-350, Alexa Fluor 488, CY3, FAM, VIC, TET, CALGold540, JOE, HEX, CALFluorOrange560, TAMRA, CALFluorRed590, ROX, CALFluorRed610, TexasRed, CALFluorRed635, Quasar670, CY5, CY5.5, LC RED640 or Quasar705.

In the present invention, the fluorophores are modified at the 5' ends of the detection probes specified at the different kinds of *Candida* and are the same fluorophores. When the same fluorophore is modified, the primer probe combination designed in the present invention can perform detection and analysis in the same fluorescence detection channel; according to different melting temperatures of the double-stranded probe formed by the detection probe and the complementary probe, melting curve analysis is conducted after the real-time fluorescent quantitative PCR amplification reaction; whether one or more of five kinds of pathogenic *Candida* exist in the to-be-detected samples is judged according to melting point changes of the Taqman probe; and the *Candida* bacteria seeds in the to-be-detected samples are identified by a Tm value-bacteria seed standard comparison table, i.e., the multiple single-color real-time fluorescent quantitative PCR reaction is carried out. When the different fluorophores are modified, the multiple multicolor real-time fluorescent quantitative PCR reaction can be carried out. In addition, a certain one of the five kinds of *Candida* can be detected alone, i.e., the single single-color real-time fluorescent quantitative PCR reaction can be carried out.

Preferably, the quencher modified at the 3' end of the detection probe includes any one or a combination of at least two of TAMRA, DABCYL, BHQ-1, BHQ-2, BHQ-3 or Eclipse.

With respect to the different kinds of *Candida,* the used fluorophores and quenchers may be the same or different, but different fluorophores need different fluorescence detection channels. Moreover, spectral ranges of excited light and absorbed light of the different selected fluorophores should avoid interference. Preferably, the fluorophores and quenchers modified by the probe for detecting different kinds of the *Candida* are in the same group.

A combination of the fluorophore and the quencher may be typically and non-finitely the fluorophore FAM and the quencher TAMRA, the fluorophore HEX and the quencher BHQ-1, the fluorophore ROX and the quencher BHQ-2, and the fluorophore CY5 and the quencher BHQ-3, preferably the fluorophore FAM and the quencher TAMRA.

Preferably, the 3' end of the complementary probe is modified with the quencher.

Preferably, the quencher modified at the 3' end of the complementary probe includes any one or a combination of at least two of TAMRA, DABCYL, BHQ-1, BHQ-2, BHQ-3 or Eclipse.

Preferably, the complementary probe and the detection probe have the same quencher. The quencher of the probe may be different from the quencher of the complementary sequences, but spectral bands of the fluorophores need to be matched.

In the present invention, the quencher can be selectively modified at the 3' end of the complementary probe. The detection probe is complementary to the target sequence. During design, the detection probe is labeled with the fluorophore at the 5' end and labeled with the quencher at the 3' end. The 3' end of the complementary probe may be designed to include the quencher, may also be designed without the quencher. When the 3' end of the complementary probe is not provided with the quencher, a fluorescence value is decreased along with unwinding of strands of the detection probe and the complementary probe during temperature rise. The reason of such a phenomenon is as follows: a linear single-stranded double-labeled oligonucleotide in the solution is of random coil: two ends of the oligonucleotide are close to each other occasionally, and thus energy of the fluorophore is absorbed by the quencher. However, when the detection probe is combined with the complementary probe, two ends of the probe are forced to separate by a labeled heterozygote, and interaction between labels at the two terminals is destructed, thereby changing the fluorescence value. If the complementary probe is labeled with the quencher at the 3' end, the fluorescence value is decreased by hybridization between the probes. Moreover, when the temperature rises to cause double-stranded denaturation, the fluorescence value is increased, which is because the quencher and the fluorophore are closer to each other due to formation of the probe heterozygote and the fluorescence signal is effectively quenched.

In a second aspect, the present invention provides a kit for detecting separate *Candida* types. The kit includes the primer probe combination in the first aspect.

Preferably, the kit further includes negative quality control, positive quality control and auxiliary reagents.

Preferably, the negative quality control is plasmids containing arabidopsis DNA fragments; and a nucleotide sequence of the arabidopsis DNA fragments is shown as SEQ ID NO.24.

Preferably, the positive quality control is respectively a plasmid containing *Candida albicans* DNA fragments, a plasmid containing *Candida tropicalis* DNA fragments, a plasmid containing *Candida parapsilosis* DNA fragments, a plasmid containing *Candida krusei* DNA fragments, and a plasmid containing *Candida glabrata* DNA fragments.

In the present invention, both the negative quality control and the positive quality control adopt a common plasmid pMD19 in the field. It should be indicated that, any vector meeting plasmid construction of the negative quality control and the positive quality control may be used for replacing the pMD19. A special limitation is not given herein.

Preferably, nucleotide sequences of the *Candida albicans* DNA fragments, the *Candida tropicalis* DNA fragments, the *Candida parapsilosis* DNA fragments, the *Candida krusei* DNA fragments and the *Candida glabrata* DNA fragments are respectively shown as SEQ ID NO.25, SEQ ID NO.26, SEQ ID NO.27, SEQ ID NO.28 and SEQ ID NO.29.

Preferably, the auxiliary reagents include a Taq enzyme, dNTP, MgCl₂, DMSO and a buffer solution.

Preferably, the buffer solution includes Tris-HCl and KCl.

Preferably, a concentration of the Tris-HCl in the buffer solution is 15-20 mM, e.g., 15 mM, 16 mM, 17 mM, 18 mM, 19 mM or 20 mM.

Preferably, a concentration of the KCl in the buffer solution is 100-200 mM, e.g., 100 mM, 110 mM, 120 mM, 130 mM, 140 mM, 150 mM, 160 mM, 170 mM, 180 mM, 190 mM or 200 mM.

In a third aspect, the present invention provides a method for detecting separate *Candida* types by utilizing the kit in the second aspect. The method includes the following steps:
(1) extracting sample DNA;
(2) adding a Taq enzyme, dNTP, MgCl₂, DMSO, a buffer solution and the primer probe combination of five kinds of Candida according to the appended claims into the sample DNA in the step (1); and
(3) carrying out a real-time fluorescent PCR reaction.

After the step (3), the method further comprises a judgement step as follows:
(a) if the Ct value of the detection channel is not greater than 35, the result is positive; and if the Ct value of the detection channel is greater than 35, the result is negative; and
(b) the positive result is further analyzed; by virtue of melting curve analysis, corresponding annealing temperature values Tm of the product and five kinds of positive quality control are contrasted; and if the Tm values are the same, to-be-detected samples are judged to contain Candida types corresponding to the positive quality control.

Preferably, a final concentration of the DNA sample in the step (2) is 0.1 pg-10 ng, e.g., 0.1 pg, 0.5 pg, 1 pg, 2 pg, 3 pg, 4 pg, 5 pg, 10 pg, 20 pg, 30 pg, 40 pg, 50 pg, 60 pg, 70 pg, 80 pg, 90 pg, 100 pg, 200 pg, 300 pg, 400 pg, 500 pg, 600 pg, 700 pg, 800 pg, 900 pg, 1 ng, 2 ng, 3 ng, 4 ng, 5 ng, 6 ng, 7 ng, 8 ng, 9 ng or 10 ng.

Preferably, a final concentration of the Taq enzyme in the step (2) is 0.5-5 U, e.g., 0.5 U, 0.8 U, 1 U, 1.2 U, 1.5 U, 1.8 U, 2 U, 2.5 U, 3 U, 3.5 U, 4 U, 4.5 U or 5 U.

Preferably, a final concentration of the dNTP in the step (2) is 0.1-5 M, e.g., 0.1 M, 0.3 M, 0.5 M, 0.8 M, 1M, 1.2 M, 1.5 M, 1.8 M, 2 M, 2.5 M, 2.8 M, 3 M, 3.5 M, 4M, 4.5 M, 4.8 M or 5 M.

Preferably, a volume percentage of the DMSO in the step (2) is 1-8%, e.g., 1%, 2%, 3%, 4%, 5%, 6%, 7% or 8%, preferably 5%.

Preferably, a final concentration of specific primers of the five kinds of *Candida* in the step (2) is 50-500 nM, e.g., 50 nM, 60 nM, 70 nM, 80 nM, 90 nM, 100 nM, 120 nM, 150 nM, 180 nM, 200 nM, 250 nM, 300 nM, 350 nM, 400 nM, 450 nM or 500 nM.

Preferably, a final concentration of a detection probe of the five kinds of *Candida* in the step (2) is 50-500 nM, e.g., 50 nM, 60 nM, 70 nM, 80 nM, 90 nM, 100 nM, 120 nM, 150 nM, 180 nM, 200 nM, 250 nM, 300 nM, 350 nM, 400 nM, 450 nM or 500 nM.

Preferably, a final concentration of a complementary probe of the five kinds of *Candida* in the step (2) is 50-500 nM, e.g., 50 nM, 60 nM, 70 nM, 80 nM, 90 nM, 100 nM, 120 nM, 150 nM, 180 nM, 200 nM, 250 nM, 300 nM, 350 nM, 400 nM, 450 nM or 500 nM.

In the present invention, the specific primers specified at each kind of *Candida* in the PCR reaction system have the consistent final concentration; according to a combination ratio 1:1:1:1:1 of the five pairs of primers, the final concentration range is 50-500 nM; and the primer design in the present invention is designed to meet amplification efficiency consistency.

In the present invention, the detection probes specified at each kind of *Candida* in the PCR reaction system have the consistent final concentration; and according to a combination ratio 1:1:1:1:1 of the five probes, the final concentration range is 50-500 nM.

In the present invention, the complementary probes specified at each kind of *Candida* in the PCR reaction system have the consistent final concentration; and according to a combination ratio 1:1:1:1:1 of the five probes, the final concentration range is 50-500 nM.

Preferably, conditions of the real-time fluorescent PCR reaction in the step (3) are as follows:
(1') pre-degeneration at 90-98°C for 1-10 min, and 1 cycle;
(2') degeneration at 90-98°C for 10 s; extension at 55-60°C for 35-45 s; and 40-50 cycles; and
(3') heating at 35-97°C; and 1 cycle.

Preferably, before the step (1'), the reaction further includes a preheating process: 50°C for 2 min; and 1 cycle.

Preferably, the real-time fluorescent PCR reaction in the step (3) specifically includes the following steps:
(1") preheating at 50°C for 2 min, and conducting 1 cycle;
(2") performing pre-degeneration at 95°C for 10 min, and conducting 1 cycle;
(3") performing degeneration at 95°C for 10 s; performing extension at 58°C for 40 s; acquiring a fluorescence signal during extension; and conducting 45 cycles;
(4") maintaining the temperature at 95°C for 5 s and at 35°C for 1 min; raising the temperature to 97°C at 0.2°C per second; continuously acquiring the fluorescence signal for 5 times per °C.

In a fourth aspect, the present invention provides an application of the primer probe combination according to any of the appended claims and/or the kit of the invention in detection of separate *Candida* types or preparation of a reagent for detection of separate *Candida* types.

Compared with the prior art, the present invention has beneficial effects as follows:
(1) The primer probe combination in the present invention has high specificity and sensitivity, and can simultaneously detect the *Candida albicans, Candida tropicalis, Candida parapsilosis, Candida krusei* and *Candida glabrata* and distinguish specific bacteria seeds.
(2) The primers designed for the *Candida albicans*, *Candida tropicalis*, *Candida parapsilosis*, *Candida krusei* and *Candida glabrata* in the present invention meet the amplification efficiency consistency while meeting ordinary amplification requirements, so that the detection system is more stable and excellent in detection specificity, thereby avoiding mutual affects among the bacteria seeds due to amplification efficiency differences.
(3) The detection method in the present invention is high in sensitivity, can rapidly detect the *Candida albicans*, *Candida tropicalis*, *Candida parapsilosis*, *Candida krusei* and *Candida glabrata* in combination with the specific primer probe combination, can identify and distinguish different *Candida* types in the same fluorescence detection channel simultaneously, and can also distinguish the specific target sequences of the multiple *Candida* types in the same fluorescence channel in accordance with the melting point change of the Taqman probe.
(4) Melting curve analysis of the oligonucleotide probe may be directly conducted after the real-time fluorescent PCR amplification reaction, without an open-tube operation, and may also be conducted in a real-time fluorescent PCR instrument after ordinary PCR amplification.
(5) The melting curve analysis of the oligonucleotide probe belongs to non-expendable testing; and after the analysis is ended, the samples are maintained in a state after the PCR and can be repeatedly analyzed.

### DESCRIPTION OF THE DRAWINGS

Fig. 1 is a melting curve of a product detected by taking plasmids containing DNA fragments of *Candida albicans*, *Candida tropicalis*, *Candida parapsilosis*, *Candida krusei* and *Candida glabrata* as a template respectively;
Fig. 2 is an amplification curve corresponding to a standard plasmid of different concentrations containing DNA fragments of *Candida albicans* in Embodiment 4;
Fig. 3 is a melting curve corresponding to a standard plasmid of different concentrations containing DNA fragments of *Candida albicans* in Embodiment 4;
Fig. 4 is an amplification curve chart of different concentration gradients of *Candida albicans* in Embodiment 5; and
Fig. 5 is a linear relation diagram between different concentrations of *Candida albicans* and a Ct value in Embodiment 5.

### DETAILED DESCRIPTION OF PREFERRED EMBODIMENTS

To further describe technological means taken in the present invention and effects of the technological means, technical solutions in the present invention will be further described below through specific embodiments. However, the present invention is not limited in the scope of the embodiments.

### Embodiment 1 Assembly of kit

Nucleotide sequences of specific primers, detection probes and complementary probes for *Candida albicans, Candida tropicalis, Candida parapsilosis*, *Candida krusei* and *Candida glabrata* are shown in Table 1.

**Table 1**

| Primer name | SEQ ID NO. | Nucleotide sequence 5'-3' |
|---|---|---|
| *Candida albicans* forward primer | 1 | TGTTTGAGCGTCGTTTCT |
| *Candida albicans* reverse primer | 2 | AAGTTCAGCGGGTAGTCC |
| *Candida tropicalis* forward primer | 3 | GCCTGTTTGAGCGTCGTTTC |
| *Candida tropicalis* reverse primer | 4 | CGCCTTACCACTACCGTCTT |
| *Candida parapsilosis* forward primer | 5 | CTGTTTGAGCGTCGTTTCT |
| *Candida parapsilosis* reverse primer | 6 | TAAGTTCAGCGGGTAGTCC |
| *Candida krusei* forward primer | 7 | AGTACTACACTGCGTGAGCG |
| *Candida krusei* reverse primer | 8 | TGCGAGAACCAAGAGATCCG |
| *Candida glabrata* forward primer | 9 | AACGCAGCGAAATGCGATAC |
| *Candida glabrata* reverse primer | 10 | GGAATACCAGAGGGCGCAAT |
| *Candida albicans* detection probe | 11 | ATCACTGTACTTGTTCGCTATCGGT |
| *Candida tropicalis* detection probe | 12 | TTTCCACTCATTGGTACAAACTCCA |
| *Candida parapsilosis* detection probe | 13 | ACGCACACTCCCAGGTCTTT |
| *Candida krusei* detection probe | 14 | TCCTCCGTCTATGCTCCGTTACCTGTT |
| *Candida glabrata* detection probe | 15 | AAACGCTTATTTTGCTAGTGGCCA |
| *Candida albicans* complementary probe | 16 | TAGTGACATGAACAAGCGATAACCA |
| *Candida tropicalis* complementary probe | 17 | AAAGGCGAGAAACCAAGTTAGAGGT |
| *Candida parapsilosis* complementary probe | 18 | TGCGAGTGAGGGACCAGAAA |
| *Candida krusei* complementary probe | 19 | AGGATGCACATACGAGACAATGGACAA |
| *Candida glabrata* complementary probe | 20 | TTTGCGAATAAAACCATCACCGGT |
| Forward primer of internal reference primer | 21 | CTGTGAATGCCATTTCTC |
| Reverse primer of internal reference primer | 22 | ACACCTCCTTTGGAATAG |
| Internal reference probe | 23 | CCTAACCTACCCGCCTTGGATATT |

Negative quality control: the negative quality control is a plasmid containing arabidopsis DNA fragments; a nucleotide sequence of the arabidopsis DNA fragments is shown as SEQ ID NO.24; and the specific sequence is as follows:

CATGATTCAGCCAACATAACCCGACCCGTACAATTCTATTACGCGT
CTCCGGCGACTGACTATACTTGCCGTTCGGAGTTAGGGTTTTACTCCGAGA
GAAAATTGAGTCAGCGATGAATCCGTTGACAAGGTGAAGAAGACGCAGA
GCATTAACGCGAAAGAATTAGATCTAGGGATTTACGACGAAGCATCTTGGC
ACGCCAAGTACAAAGATTCAGCCTACGTTTACGTCGGAGAATTACCTTACG
ATCTCACGGAGGGTGACCTCCTCGCCGTTTTCTCACAATATGGTGAAGTTG
TTGATTTGAATCTTGTTCGAGATAAAGGAACTGGGAGATCAAAAAGATTTG
CGTTTGTTGCTTATGAAGATCAGAGAAGTACTAATCTTGCTGTTGGATAATA
AAAGTGGAGCATTGTGGTAAATACTTAAAGAGAGAAGAGGAAGATGAAG
AGACGAAGCAGAAGAAGAGAGAAGCTCCGTGGTGTTTGCAGAGCTTTTC
AGAGAAAGGAGTGTACTCGTGGAGATTCTTGCAAATTTTCTCACGATGAA
AATAGAGCTGCCAATACCGGGTGGGGTCACGAAGATCGTAGAAGTTCCAA
GTGAGAGATCAAGTAAGTAAATACCATAATGATGTAGAGGATAAATAGGGA
TTGTTCATATTGATATCCAAGTGGTAATGCTCTACATTGAAGAATGGTTTCT
AAGTTGTAGCTAGAATCTAATGGAAAATGTGATTTATTGACCATTAGCTTAC
ACACCGGTGTTTTGCTCTGTATATGTCGATCTTATTTTCAGTGTTCACCTTTA
C.

Positive quality control: a plasmid containing *Candida albicans* DNA fragments, a plasmid containing *Candida tropicalis* DNA fragments, a plasmid containing *Candida parapsilosis* DNA fragments, a plasmid containing *Candida krusei* DNA fragments, and a plasmid containing *Candida glabrata* DNA fragments.

A nucleotide sequence of the *Candida albicans* DNA fragments is shown as SEQ ID NO.25, and is specifically as follows:

ATATCAATAAGCGGAGGAAAAGAAACCAACAGGGATTGCCTCAGT
AGCGGCGAGTGAAGCGGCAAAAGCTCAAATTTGAAATCTGGCGTCTTTGG
CGTCCGAGTTGTAATTTGAAGAAGGTATCTTTGGGCCCGGCTCTTGTCTAT
GTTCCTTGGAACAGGACGTCACAGAGGGTGAGAATCCCGTGCGATGAGAT

GACCCGGGTCTGTGTAAAGTTCCTTCGACGAGTCGAGTTGTTTGGGAATG
CAGCTCTAAGTGGGTGGTAAATTCCATCTAAAGCTAAATATTGGCGAGAGA
CCGATAGCGAACAAGTACAGTGATGGAAAGATGAAAAGAACTTTGAAAA
GAGAGTGAAAAAGTACGTGAAATTGTTGAAAGGGAAGGGCTTGAGATCA
GACTTGGTATTTTGCATGCTGCTCTCTCGGGGGCGGCCGCTGCGGTTTACC
GGGCCAGCATCGGTTTGGAGCGGCAGGATAATGGCGGAGGAATGTGGCAC
GGCTTCTGCTGTGTGTTATAGCCTCTGACGATACTGCCAGCCTAGACCGAG
GACTGCGGTTTTTACCTAGGATGTTGGCATAATGATCTTAAGTCGC.

A nucleotide sequence of the *Candida tropicalis* DNA fragments is shown as SEQ ID NO.26, and is specifically as follows:

AAAAGAAACCAACAGGGATTGCCTTAGTAGCGGCGAGTGAAGCG
GCAAAAGCTCAAATTTGAAATCTGGCTCTTTCAGAGTCCGAGTTGTAATTT
GAAGAAGGTATCTTTGGGTCTGGCTCTTGTCTATGTTTCTTGGAACAGAAC
GTCACAGAGGGTGAGAATCCCGTGCGATGAGATGATCCAGGCCTATGTAA
AGTTCCTTCGAAGAGTCGAGTTGTTTGGGAATGCAGCTCTAAGTGGGTGG
TAAATTCCATCTAAAGCTAAATATTGGCGAGAGACCGATAGCGAACAAGTA
CAGTGATGGAAAGATGAAAAGAACTTTGAAAAGAGAGTGAAAAAGTACG
TGAAATTGTTGAAAGGGAAGGGCTTGAGATCAGACTTGGTATTTTGTATGT
TACTTCTTCGGGGGTGGCCTCTACAGTTTATCGGGCCAGCATCAGTTTGGG
CGGTAGGAGAATTGCGTTGGAATGTGGCACGGCTTCGGTTGTGTGTTATAG
CCTTCGTCGATACTGCCAGCCTAGACTGAGGACTGCGGTTTATACCTAGGA
TGTTGGCATAATGATCTTAAGTCGCCCGTCTTGAAACACGGACCAAGGAG
TCTAACGTCTATGCGAGTGTTTGGGTGTAAAACCCGTACGCGTAATGAAAG
TGAACGTAGGTGGGGGCCCGTATGGGTGCACCATCGACCGATCCTGATGT
CTTCGGATGGATTTGAGTAAGAGCATAGCTGTTGGGACCCGAAAGATGGT
GAACTATGCCTGAATAGGGTGAAGCCAGAGGAAACTCTGGTGGAGGCTCG
TAGCGGTTCTGACGTGCAAATCGATCGTCGAATTTGGGTATAGGGGCGAAA
GACTAATCGACCC.

A nucleotide sequence of the *Candida parapsilosis* DNA fragments is shown as SEQ ID NO.27, and is specifically as follows:

GCATATCAATAAGCGGAGGAAAAGAAACCAACAGGGATTGCCTTA
GTAGCGGCGAGTGAAGCGGCAAAAGCTCAAATTTGAAATCTGGCACTTTC
AGTGTCCGAGTTGTAATTTGAAGAAGGTATCTTTGGGTCTGGCTCTTGTCT
ATGTTTCTTGGAACAGAACGTCACAGAGGGTGAGAATCCCGTGCGATGAG

ATGTCCCAGACCTATGTAAAGTTCCTTCGAAGAGTCGAGTTGTTTGGGAAT
GCAGCTCTAAGTGGGTGGTAAATTCCATCTAAAGCTAAATATTGGCGAGAG
ACCGATAGCGAACAAGTACAGTGATGGAAAGATGAAAAGAACTTTGAAA
AGAGAGTGAAAAAGTACGTGAAATTGTTGAAAGGGAAGGGCTTGAGATC
AGACTTGGTATTTTGTATGTTACTCTCTCGGGGGTGGCCTCTACAGTTTACC
GGGCCAGCATCAGTTTGAGCGGTAGGATAAGTGCAAAGAAATGTGGCACT
GCTTCGGTAGTGTGTTATAGTCTTTGTCGATACTGCCAGCTTAGACTGAGG
ACTGCGGCTTCGGCCTAGGATGTTGGCATAATGATCTTAAGTCGCCCGTCT
TGAAACACGGACC.

A nucleotide sequence of the *Candida krusei* DNA fragments is shown as SEQ ID NO.28, and is specifically as follows:

TTTCATGCGCTTGATTTTTCAAGCCAACCATCGAAAGGGAATCCGG
TTAAAATTCCGGAACTTGGATATGGATTCTTCACGGCAACGTAACTGAATG
TGGAGACGTCGGCGTGAGCCCTGGGAGGAGTTATCTTTTCTTCTTAACAG
CTTATCACCCTGGAATTGGTTTATCCGGAGATGGGGTCTTATGGCTGGAAG
AGCGCGGTAATTTTGCCGCGTCCGGTGCGCTCACGACGGTCCTTGAAAAT
CCACAGGAAGGAATAGTTTTCATGCCAAGTCGTACTCATAACCGCAGCAG
GTCTCCAAGGTTAACAGCCTCTAGTTGATAGAATAATGTAGATAAGGGAAG
TCGGCAAAATAGATCCGTAACTTCGGGATAAGGATTGGCTCTAAGGATCGG
GTGGTTTGGGCCTTGCGTAGAAGTGGTGGTGACTGGCGGCGGGCTGCTTT
CGGGCGGACTGCTGTTGGACGTCGCTATAGACACACTTGGTAGGCATTTAT
GTCGTCCGGATCACGCTTAACGATCAACTTAGAACTGGTACGGACAAGGG
GAATCTGACTGTCTAATTAAAACATAGCATTGTGATGGTCAGAAAGTGATG
TTGACACAATGTGATTTCTGCCCAGTGCT.

A nucleotide sequence of the *Candida glabrata* DNA fragments is shown as SEQ ID NO.29, and is specifically as follows:

GCCTTAGTAACGGCGAGTGAAGCGGCAAAAGCTCAAATTTGAAAT
CTGGTACCTTTGGTGCCCGAGTTGTAATTTGGAGAGTACCACTTTGGGACT
GTACTTTGCCTATGTTCCTTGGAACAGGACGTCATGGAGGGTGAGAATCCC
GTGTGGCGAGGTGTGTCAGTTCTTTGTAAAGGGTGCTCGAAGAGTCGAGT
TGTTTGGGAATGCAGCTCTAAGTGGGTGGTAAATTCCATCTAAAGCTAAAT
ACAGGCGAGAGACCGATAGCGAACAAGTACAGTGATGGAAAGATGAAAA
GAACTTTGAAAAGAGAGTGAAAAAGTACGTGAAATTGTTGAAAGGGAAG
GGCATTTGATCAGACATGGTGTTTTGCGCCTTGCCTCTCGTGGGGGGACTC

TCGCAGCTCACTGGGCCAGCATCGGTTTTGGCGGCCGGAAAAAACCTAGG
GAATGTGGCTCTGCCTCGGTGTAGAGTTATAGCCCTGGGGAATACGGCCAG
CCGGGACCGAGGACTGCGATTTGTATCTAGGATGCTGGCATAATGGTTATAT
GCCGCCCGTCTTGAAACACGGACCAAGGAGTCTAACGTCTATGCGAGTGT
TTGGGTGTTAAACCCGTACGCGTAATGAAAGTGAACGTAGGTTGGGCCCC
TGGGGGGTGCACAATCGACCGATCCTGATGTCTTCGGATGGATTTGAGTAA
GAGCATAGCTGTTGGGACCCGAAAGATGGTGAACTATGCCTGAATAGGGT
GAAGCCAGAGGAAACTCTGGTGGAGGCTCGTAGCGGTTCTGACGTGCAA
ATCGATCGTCGAATTTGGGTATAGGGGCGAAAGACTAATCGAACCATCTAG
TAGCTGGTTCCTGCCGAAGTT.

Auxiliary reagents: a Taq enzyme, dNTP, MgCl₂, DMSO and a buffer solution.

Centrifuge tubes filled with the above components and instructions were packed in the kit.

### Embodiment 2 Detection of separate Candida types

Separate *Candida* types were detected by the kit in Embodiment 1, and the detection method includes the following steps:
(1) sample DNA was extracted;
(2) a Taq enzyme, dNTP, MgCl₂, DMSO, a buffer solution, and specific primers, detection probes and complementary probes for five kinds of *Candida* were added into the sample DNA in the step (1); and preparing a system according to Table 2;

**Table 2**

| Component | Dosage |
|---|---|
| TaqPolymerase | 2 U |
| dNTP | 2 M |
| Primers (5 pairs) | 200 nM*10 |
| Probes (5) | 100 nM*5 |
| MgCl₂ | 3 mM |
| Template | 1 µL |
| DMSO | 5% |
| Total | 25 µL |

(3) the system in the step (2) was put into a real-time fluorescent PCR instrument; and a PCR amplification reaction was carried out, wherein specific conditions were as follows:
(1") preheating was performed at 50°C for 2 min, and 1 cycle was conducted;
(2") pre-degeneration was performed at 95°C for 10 min, and 1 cycle was conducted;
(3") degeneration was performed at 95°C for 10 s; extension was performed at 58°C for 40 s; a fluorescence signal was acquired during extension; and 45 cycles were conducted; and
(4") the temperature was maintained at 95°C for 5 s and at 35°C for 1 min; the temperature was raised to 97°C at 0.2°C per second; the fluorescence signal was continuously acquired for 5 times per °C.

A fluorescence detection channel in the step (4") was FAM; PCR procedures were operated; and a file was saved;
(4) Result determination:
1) Determination of kit effectiveness:
Negative quality control group effectiveness: the negative quality control group had no typical amplification curve or no Ct value in the channel FAM, otherwise reagent pollution may exist; determination should be performed after a pollution source was removed;
Positive quality control group effectiveness: all the positive quality control groups had typical amplification curves in the channel FAM; Ct values of the positive quality control were regulated to 20 by regulating thresholds after confirmation; and the Ct values were taken as thresholds of the to-be-detected samples;

2) Effectiveness determination of detected samples:
If the Ct value existed, it was indicated that the added DNA had a normal amount;
3) Determination of separate *Candida* types:
After the products were subjected to cyclic PCR amplification, DNA sequences of different *Candida* types had different melting temperatures; instrument calibration was performed by virtue of five plasmids in the positive quality control; and the *Candida* types were detected by Roche 480 in the present invention so as to obtain a product annealing temperature table (Table 3) corresponding to *Candida albicans, Candida tropicalis, Candida parapsilosis, Candida krusei* and *Candida glabrata.* It should be indicated that, any instrument meeting the real-time fluorescent PCR was available. A specific limitation was not needed herein. A purpose of calibration was to eliminate specific annealing temperature differences brought by different instruments.

**Table 3 Comparison table of annealing temperature Tm of different Candida types**

| *Candida* types | Melting temperature (Tm value) °C |
|---|---|
| *Candida albicans* | 63 |
| *Candida tropicalis* | 40 |
| *Candida parapsilosis* | 52 |
| *Candida krusei* | 46 |
| *Candida glabrata* | 58 |

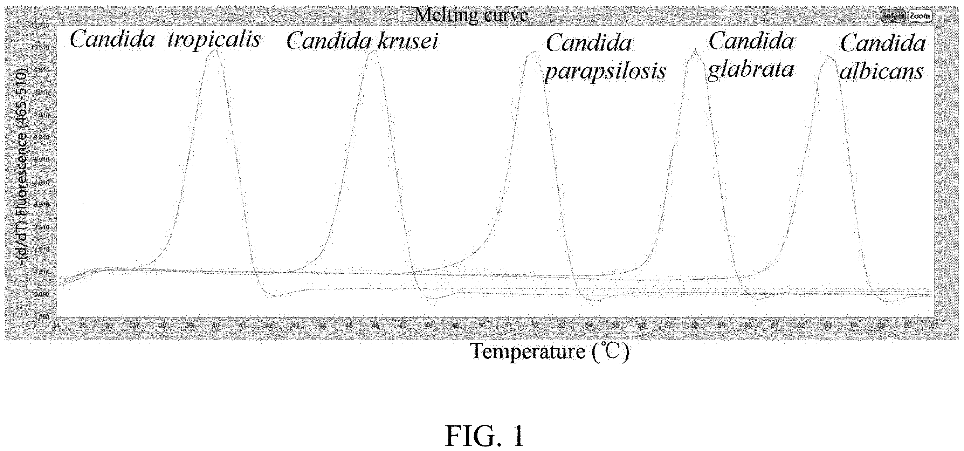

It can be seen from Table 2 that, the Tm values of the products of different *Candida* types have greater differences, which are up to 4°C or higher. Thus, the *Candida* types are conveniently distinguished; the specificity is excellent; the sensitivity is high; and specific measured values that are not limited to the PCR instrument may have slight deviation, but the Tm values among the *Candida* types are relatively stable. By detecting the positive quality control, the Tm value comparison table applicable to any machine may be obtained; and a melting curve chart corresponding to the positive quality control is shown as Fig. 1.

### Embodiment 3 Specificity detection and analysis

DNA of *Cryptococcus neoformans*, *Cryptococcus gattii*, *Aspergillus fumigates, Aspergillus flavus, Aspergillus terreus, Aspergillus niger, Aspergillus nidulans, Aspergillus versicolor, Saccharomyces cerevisiae, Penicillium marneffei, Candida lusitaniae, Candida guilliermondii, Bordetella pertussis, Haemophilus influenza*, *Pseudomonas aeruginosa*, *Staphylococcus aureus* and *Streptococcus pneumonia* was respectively extracted, and then detected by the kit in Embodiment 1 of the present invention according to the method in Embodiment 2. Results were shown in Table 4 below:

**Table 4**

| Name of strains | | Detected Ct value | Detected Tm value |
|---|---|---|---|
| *Cryptococcus neoformans* | | - | - |
| | *Cryptococcus gattii* | - | - |
| | *Aspergillus fumigatus* | - | - |
| | *Aspergillus flavus* | - | - |
| | *Aspergillus terreus* | - | - |
| | *Aspergillus niger* | - | - |
| | *Aspergillus nidulans* | - | - |
| | *Aspergillus versicolor* | - | - |
| *Saccharomyces cerevisiae* | | - | - |
| | *Penicillium marneffei* | - | - |
| | *Candida lusitaniae* | - | - |
| | *Candida guilliermondii* | - | - |
| | *Bordetella pertussis* | - | - |
| | *Haemophilus influenzae* | - | - |
| | *Pseudomonas aeruginosa* | | |
| | *Staphylococcus aureus* | - | - |
| *Streptococcus pneumoniae* | | - | - |

*Cryptococcus, Aspergillus* and *Candida* are clinically common fungi causing invasive fungal diseases. The above bacteria are common bacteria seeds causing bacterial infections in respiratory tracts and the like. It can be seen from Table 4 that, in presence of nucleic acids of the above fungi or bacteria, any amplification reaction is not carried out in the detection system in the present invention, and any specific melting curve or Tm value is not detected, which indicates that the present invention has excellent specificity.

### Embodiment 4 Sensitivity detection and analysis

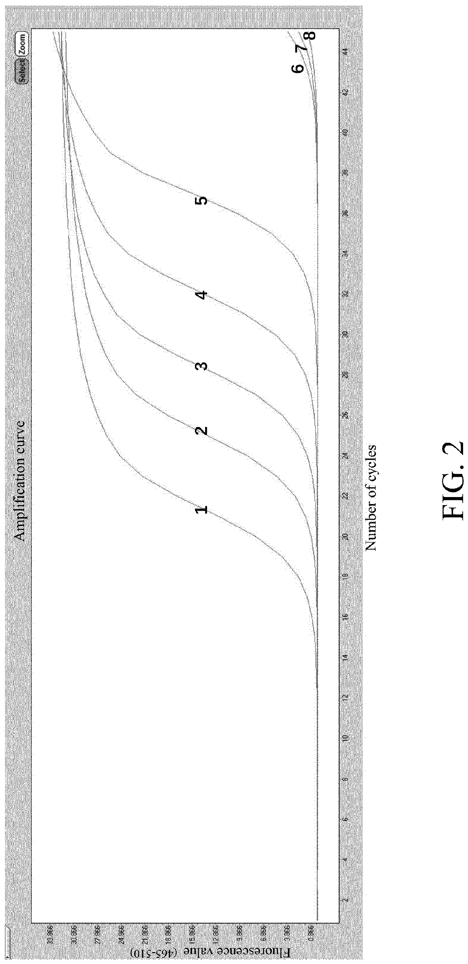

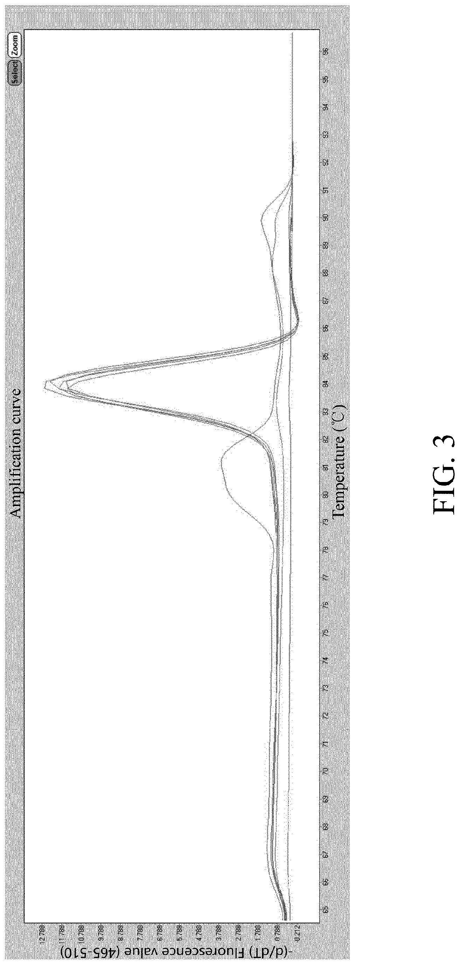

The sensitivity of plasmid standard substances of target sequences of five kinds of *Candida* of different concentrations was detected by a melting curve method; the plasmid standard substances (Shanghai Sangon Synthesis) of target sequences of synthetic *Candida albicans, Candida tropicalis, Candida parapsilosis, Candida krusei* and *Candida glabrata* were diluted into 10⁶ copies, 10⁵ copies, 10⁴ copies, 10³ copies, 10² copies, 10¹ copies and 10⁰ copies. The plasmid standard substances were taken as templates, detected by the kit in Embodiment 1 and the method in Embodiment 2 by utilizing a Roche 480II fluorescent quantitative PCR instrument, and then subjected to melting curve analysis. By taking the *Candida albicans* as an example, amplification curves of different concentrations were shown as Fig. 2, wherein curves 1-8 respectively corresponded to 10⁶ copies, 10⁵ copies, 10⁴ copies, 10³ copies, 10² copies, 10¹ copies, 10⁰ copies and negative control; and the melting curve was shown as Fig. 3.

It can be seen from the amplification curve in Fig. 2, in presence of the plasmid standard substances of 10⁶ copies-10² copies, an excellent signal is given in the present invention; in presence of the plasmid standard substances of 10¹ copies-10⁰ copies, a fluorescence signal cannot be detected; and in absence of the target sequence, the fluorescence signal cannot be detected in the present invention. Through the melting curve in Fig. 3, it is indicated that, in presence of the plasmid standard substances of 10⁶ copies-10² copies, unique and consistent melting points is formed, and a single peak in the figure is formed; in presence of the plasmid standard substances of 10¹ copies-10⁰ copies, a unique and consistent single peak cannot be formed; and in absence of the target sequence, there is no single peak. Therefore, the sensitivity of the plasmid standard substances for detecting the target sequences of the *Candida* by the melting curve method in the present invention may be up to 10² copies.

### Embodiment 5 Quantitative analysis

Five kinds of *Candida* were quantitatively detected, such as *Candida albicans, Candida tropicalis, Candida parapsilosis, Candida krusei* and *Candida glabrata.*

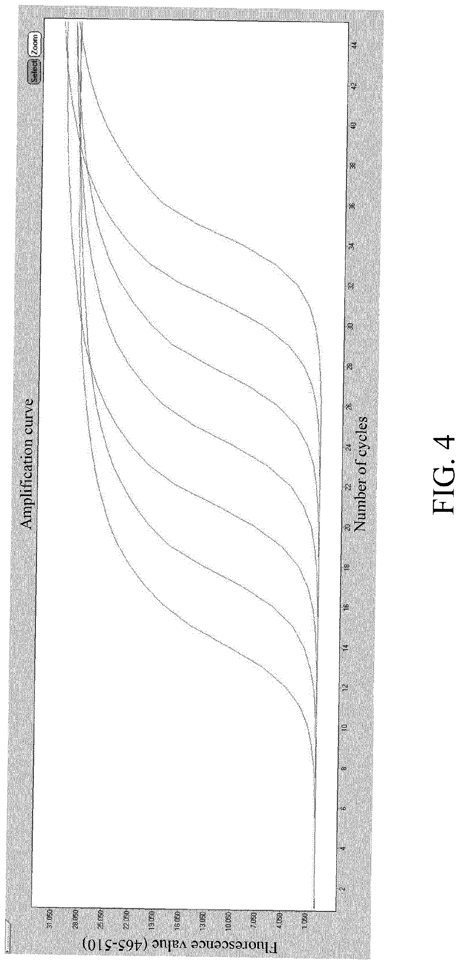

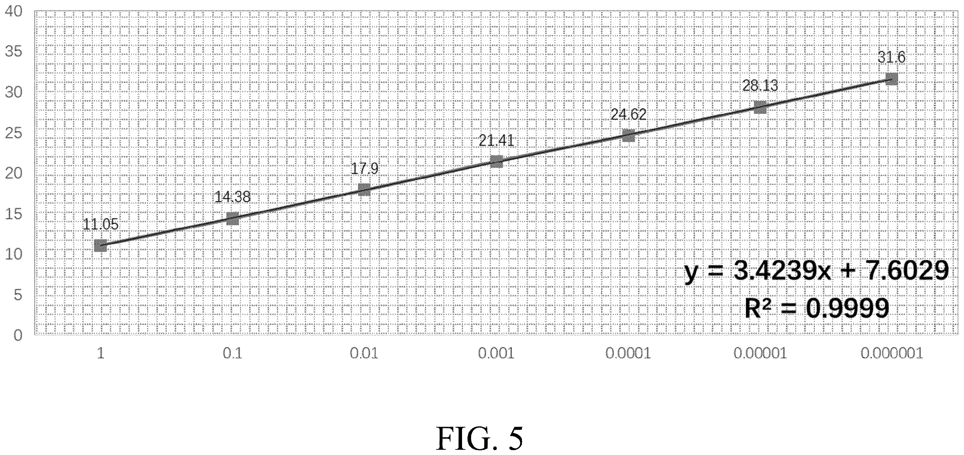

Each of the five kinds of *Candida* that is detected by the kit was equivalently and uniformly mixed at a respective concentration of 1 ng/µl, and subjected to 10-times continuous gradient dilution so as to prepare a standard curve of 7 concentration points; each concentration was subjected to 3 duplications; arabidopsis gene fragments were taken as negative control; an amplification curve was shown as Fig. 4 (taking *Candida albicans* as an example); and a linear relation between the Ct value and the concentration was shown as Fig. 5.

It can be seen from Fig. 5 that, a standard curve formed a linear equation y=3.4329x+7.6029 in a linear amplification region of the PCR reaction; R2 was equal to 0.9999; amplification efficiency of the kit was 95.9%; and a linear range was 1 ng/µl-0.000001 ng/µl. When the samples were measured, the above standard curve, the negative control and sample DNA of an unknown concentration were subjected to fluorescent PCR reactions; and when the fluorescent quantitative PCR instruments such as Roche LightCycler 480II, ABI7500 and StepOne Plus were used, a nucleic acid concentration of target genes in the samples are calculated by built-in analysis software according to the standard curve and the Ct value of the sample, thereby conducting quantitative analysis.

### Embodiment 6 Stability detection

1) A stability detection kit*-Candida albicans* was formed by utilizing the auxiliary reagents in Embodiment 1, and specific primers, a detection probe and a complementary probe for the *Candida albicans* according to components and dosages in the following table; a stability detection kit*-Candida parapsilosis* was formed by utilizing the auxiliary reagents in Embodiment 1, and specific primers, a detection probe and a complementary probe for the *Candida parapsilosis* according to components and dosages in the following table; a stability detection kit*-Candida tropicalis* was formed by utilizing the auxiliary reagents in Embodiment 1, and specific primers, a detection probe and a complementary probe for the *Candida tropicalis* according to components and dosages in the following table; a stability detection kit*-Candida krusei* was formed by utilizing the auxiliary reagents in Embodiment 1, and specific primers, a detection probe and a complementary probe for the *Candida krusei* according to components and dosages in the following table; and a stability detection kit*-Candida glabrata* was formed by utilizing the auxiliary reagents in Embodiment 1, and specific primers, a detection probe and a complementary probe for the *Candida glabrata* according to components and dosages in the following table. A separate detection system for each kind of *Candida* was shown as Table 5:

**Table 5**

| Component | Dosage |
|---|---|
| TaqPolymerase | 2 U |
| dNTP | 2 M |
| Primers (1 pair) | 200 nM*2 |
| Probe (1) | 100 nM |
| MgCl₂ | 3mM |
| Template | 1 µL |
| DMSO | 5% |
| Total | 25 µL |

2) DNA of the cultured *Candida albicans, Candida tropicalis, Candida parapsilosis*, *Candida krusei* and *Candida glabrata* was extracted;
3) Ct values of the DNA of the above strains detected by the kit in Embodiment 1 were respectively compared with Ct values of corresponding strains in 2) detected by the five stability detection kits in 1) by utilizing the method in Embodiment 2, i.e., results of the detected Ct values of the DNA of the five kinds of *Candida* in Embodiment 1 and the detected Ct values of the DNA of the five kinds of *Candida* by the stability detection kits were compared; the standard deviation and CV values of the two groups of results were analyzed and compared; and the results were shown as Table 6 as follows:

**Table 6**

| | *Candida albicans* | *Candida parapsilosis* | *Candida tropicalis* | *Candida krusei* | *Candida glabrata* |
|---|---|---|---|---|---|
| Detection kit | 23.12±0.05 | 25.56±0.06 | 21.26±0.04 | 20.76±0.05 | 19.98±0.06 |
| Stability kit | 23.13±0.03 | 25.55±0.08 | 21.25±0.05 | 20.74±0.04 | 19.97±0.08 |
| STD | 0.05 | 0.06 | 0.04 | 0.05 | 0.06 |
| CV% | <3 | <3 | <3 | <3 | <3 |

It can be seen from Table 6 that, the complex primer and probe system designed in the detection kit has the detection efficiency consistent with that of a separate system; and the detection performance in the complex system is stable.

## Claims

1. A primer probe combination for detecting separate *Candida* types, comprising specific primers, detection probes and complementary probes specified at *Candida albicans, Candida tropicalis, Candida parapsilosis, Candida krusei* and *Candida glabrata* respectively, wherein the complementary probes and the detection probes are in incomplete complementary pairing;
wherein nucleotide sequences of the specific primers of the *Candida albicans* are shown as SEQ ID NO. 1-2; nucleotide sequences of the specific primers of the *Candida tropicalis* are shown as SEQ ID NO.3-4; nucleotide sequences of the specific primers of the *Candida parapsilosis* are shown as SEQ ID NO. 5-6; nucleotide sequences of the specific primers of the *Candida krusei* are shown as SEQ ID NO. 7-8; and nucleotide sequences of the specific primers of the *Candida glabrata* are shown as SEQ ID NO. 9-10;
a nucleotide sequence of the detection probe of the *Candida albicans* is shown as SEQ ID NO. 11; a nucleotide sequence of the detection probe of the *Candida tropicalis* is shown as SEQ ID NO. 12; a nucleotide sequence of the detection probe of the *Candida parapsilosis* is shown as SEQ ID NO. 13; a nucleotide sequence of the detection probe of the *Candida krusei* is shown as SEQ ID NO. 14; and a nucleotide sequence of the detection probe of the *Candida glabrata* is shown as SEQ ID NO. 15;
a nucleotide sequence of the complementary probe of the *Candida albicans* is shown as SEQ ID NO. 16; a nucleotide sequence of the complementary probe of the *Candida tropicalis* is shown as SEQ ID NO. 17; a nucleotide sequence of the complementary probe of the *Candida parapsilosis* is shown as SEQ ID NO. 18; a nucleotide sequence of the complementary probe of the *Candida krusei* is shown as SEQ ID NO. 19; and a nucleotide sequence of the complementary probe of the *Candida glabrata* is shown as SEQ ID NO. 20;
wherein a quencher is modified at 3' end of the detection probe of each one of the *Candida albicans, Candida tropicalis, Candida parapsilosis, Candida krusei* and *Candida glabrata*; and a fluorophore is modified at 5' end of the detection probe of each one of the *Candida albicans, Candida tropicalis, Candida parapsilosis, Candida krusei* and *Candida glabrata*; the fluorophores modified at the 5' ends of the detection probes are the same.

2. The primer probe combination according to claim 1, wherein the primer probe combination further comprises an internal reference system; the internal reference system comprises internal reference primers and an internal reference probe; nucleotide sequences of the internal reference primers are shown as SEQ ID NO. 21-22; and a nucleotide sequence of the internal reference probe is shown as SEQ ID NO. 23.

3. The primer probe combination according to claim 1, wherein a quencher is modified at 3' end of the complementary probe of the *Candida albicans, Candida tropicalis, Candida parapsilosis, Candida krusei* and *Candida glabrata.*

4. The primer probe combination according to claims 1 or 3, wherein the fluorophore comprises any one or a combination of at least two of ALEX-350, Alexa Fluor 488, CY3, FAM, VIC, TET, CALGold540, JOE, HEX, CALFluorOrange560, TAMRA, CALFluorRed590, ROX, CALFluorRed610, TexasRed, CALFluorRed635, Quasar670, CY5, CY5.5, LC RED640 or Quasar705; and
the quencher comprises any one or a combination of at least two of TAMRA, DABCYL, BHQ-1, BHQ-2, BHQ-3 or Eclipse.

5. A kit for detecting separate *Candida types,* comprising the primer probe combination of any of claims 1-4.

6. The kit for detecting separate *Candida types* according to claim 5, further comprising negative quality control, positive quality control and auxiliary reagents.

7. The kit for detecting separate *Candida types* according to claim 6, wherein the negative quality control is plasmids containing arabidopsis DNA fragments; a nucleotide sequence of the arabidopsis DNA fragments is shown as SEQ ID NO.24; the positive quality control is respectively a plasmid containing *Candida albicans* DNA fragments, a plasmid containing *Candida tropicalis* DNA fragments, a plasmid containing *Candida parapsilosis* DNA fragments, a plasmid containing *Candida krusei* DNA fragments, and a plasmid containing *Candida glabrata* DNA fragments; nucleotide sequences of the *Candida albicans* DNA fragments, the *Candida tropicalis* DNA fragments, the *Candida parapsilosis* DNA fragments, the *Candida krusei* DNA fragments and the *Candida glabrata* DNA fragments are respectively shown as SEQ ID NO. 25, SEQ ID NO. 26, SEQ ID NO. 27, SEQ ID NO. 28 and SEQ ID NO. 29; the auxiliary reagents comprise a Taq enzyme, dNTP, MgCl₂, DMSO and a buffer solution; the buffer solution comprises Tris-HCl and KCl; a concentration of the Tris-HCl in the buffer solution is 15-20 mM; and a concentration of the KCl in the buffer solution is 100-200 mM.

8. A method for detecting separate *Candida* types by utilizing the kit of any one of claims 5-7 for a purpose of non-disease diagnosis, comprising the following steps:
(1) extracting sample DNA;
(2) adding a Taq enzyme, dNTP, MgCl2, DMSO, a buffer solution and the primer probe combination of five kinds of *Candida* in any one of claims 1-6 into the sample DNA in the step (1); and
(3) carrying out a real-time fluorescent PCR reaction;
after the step (3), the method further comprises a judgement step as follows:
(a) if the Ct value of the detection channel is not greater than 35, the result is positive; and if the Ct value of the detection channel is greater than 35, the result is negative; and
(b) the positive result is further analyzed; by virtue of melting curve analysis, corresponding annealing temperature values Tm of the product and five kinds of positive quality control are contrasted; and if the Tm values are the same, to-be-detected samples are judged to contain Candida types corresponding to the positive quality control.

9. The method according to claim 8, wherein in the step (2), a final concentration of the DNA sample is 0.1 pg-10 ng; a final concentration of the Taq enzyme is 0.5-5 U; a final concentration of the dNTP is 0.1-5 M; a volume percentage of the DMSO is 1-8%; a volume percentage of the DMSO is 5%; a final concentration of specific primers of the five kinds of *Candida* is 50-500 nM; a final concentration of a detection probe of the five kinds of *Candida* is 50-500 nM; and a final concentration of a complementary probe of the five kinds of *Candida* is 50-500 nM.

10. The method according to claim 8, wherein conditions of the real-time fluorescent PCR reaction in the step (3) are as follows:
(1') pre-degeneration at 90-98 °C. for 1-10 min, and 1 cycle;
(2') degeneration at 90-98 °C. for 10 s; extension at 55-60 °C. for 35-45 s; and 40-50 cycles; and
(3') heating at 35-97 °C.; and 1 cycle.

11. The method according to claims 8 or 9, wherein the real-time fluorescent PCR reaction in the step (3) specifically comprises the following steps:
(1") preheating at 50 °C. for 2 min, and conducting 1 cycle;
(2") performing pre-degeneration at 95 °C. for 10 min, and conducting 1 cycle;
(3") performing degeneration at 95 °C. for 10 s; performing extension at 58 °C. for 40 s; acquiring a fluorescence signal during extension; and conducting 45 cycles;
(4") maintaining the temperature at 95 °C. for 5 s and at 35 °C. for 1 min; raising the temperature to 97 °C. at 0.2 °C. per second; continuously acquiring the fluorescence signal for 5 times per °C.

12. An application of the primer probe combination of any one of claims 1-4 and/or the kit of any one of claims 5-7 in detection of separate *Candida* types or preparation of a reagent for detection of separate *Candida* types for a purpose of non-disease diagnosis.

## Patentansprüche

1. Primer-Sonden-Kombination zum Nachweis getrennter Candida-Typen, umfassend spezifische Primer, Nachweissonden und komplementäre Sonden, die jeweils für Candida albicans, Candida tropicalis, Candida parapsilosis, Candida krusei bzw. Candida glabrata spezifiziert sind, wobei die komplementären Sonden und die Nachweissonden in unvollständiger komplementärer Paarung vorliegen;
wobei Nukleotidsequenzen der spezifischen Primer der Candida albicans als SEQ ID NO. 1-2 gezeigt sind; wobei Nukleotidsequenzen der spezifischen Primer von Candida Tropicalis als SEQ ID NO. 3-4 gezeigt sind; wobei Nukleotidsequenzen der spezifischen Primer der Candida parapsilosis sind als SEQ ID NO. 5-6 gezeigt sind; wobei Nukleotidsequenzen der spezifischen Primer von Candida krusei als SEQ ID NO. 7-8 Gezeigt sind; wobei Nukleotidsequenzen der spezifischen Primer der Candida glabrata als SEQ ID NO. 9-10 Gezeigt sind;
wobei eine Nukleotidsequenz der Nachweissonde von Candida albicans als SEQ ID NO. 11 gezeigt ist; wobei eine Nukleotidsequenz der Nachweissonde von Candida Tropicalis als SEQ ID NO. 12 gezeigt ist; wobei eine Nukleotidsequenz der Nachweissonde der Candida parapsilosis als SEQ ID NO 13 gezeigt ist; wobei eine Nukleotidsequenz der Nachweissonde von Candida krusei als SEQ ID NO.14 gezeigt ist; wobei eine Nukleotidsequenz der Nachweissonde von Candida glabrata als SEQ ID NO. 15 gezeigt ist;
wobei eine Nukleotidsequenz der komplementären Sonde von Candida albicans als SEQ ID NO. 16 gezeigt ist; wobei eine Nukleotidsequenz der komplementären Sonde von Candida Tropicalis als SEQ ID NO. 17 gezeigt ist; wobei eine Nukleotidsequenz der komplementären Sonde der Candida parapsilosis als SEQ ID NO. 18 gezeigt ist; wobei eine Nukleotidsequenz der komplementären Sonde von Candida krusei als SEQ ID NO. 19 gezeigt ist; wobei eine Nukleotidsequenz der komplementären Sonde von Candida glabrata als SEQ ID NO. 20 gezeigt ist;
wobei ein Quencher am 3'-Ende der Nachweissonde von jedem der Candida albicans, Candida tropicalis, Candida parapsilosis, Candida krusei und Candida glabrata modifiziert ist; und ein Fluorophor am 5'-Ende der Nachweissonde von jedem der Candida albicans, Candida tropicalis, Candida parapsilosis, Candida krusei und Candida glabrata modifiziert ist; wobei die an den 5'-Enden der Nachweissonden modifizierten Fluorophore gleich sind.

2. Primer-Sonden-Kombination nach Anspruch 1, wobei die Primer-Sonden-Kombination ferner ein internes Referenzsystem umfasst; wobei das interne Referenzsystem interne Referenzprimer und eine interne Referenzsonde umfasst; wobei die Nukleotidsequenzen der internen Referenzprimer als SEQ ID NO. 21-22 gezeigt sind; und wobei eine Nukleotidsequenz der internen Referenzsonde als SEQ ID NO. 23 gezeigt ist.

3. Primer-Sonden-Kombination nach Anspruch 1, wobei ein Quencher am 3'-Ende der komplementären Sonde von Candida albicans, Candida tropicalis, Candida parapsilosis, Candida krusei und Candida glabrata modifiziert ist.

4. Primer-Sonden-Kombination nach Anspruch 1 oder 3, wobei der Fluorophor eines oder eine Kombination von mindestens zwei von ALEX-350, Alexa Fluor 488, CY3, FAM, VIC, TET, CALGold540, JOE, HEX, CALFluorOrange560, TAMRA umfasst, CALFluorRed590, ROX, CALFluorRed610, TexasRed, CALFluorRed635, Quasar670, CY5, CY5.5, LC RED640 oder Quasar705; und
wobei der Quencher umfasst einen oder eine Kombination von mindestens zwei von TAMRA, DABCYL, BHQ-1, BHQ-2, BHQ-3 oder Eclipse.

5. Kit zum Nachweis getrennter Candida-Typen, umfassend die Primer-Sonden-Kombination nach einem der Ansprüche 1 bis 4.

6. Kit zum Nachweis getrennter Candida-Typen nach Anspruch 5, weiterhin umfassend negative Qualitätskontrolle, positive Qualitätskontrolle und Hilfsreagenzien.

7. Kit zum Nachweis getrennter Candida-Typen nach Anspruch 6, wobei die negative Qualitätskontrolle Plasmide sind, die Arabidopsis-DNA-Fragmente enthalten; wobei eine Nukleotidsequenz der Arabidopsis-DNA-Fragmente als SEQ ID NO. 24 gezeigt ist, wobei die positive Qualitätskontrolle jeweils ein Plasmid ist, das Candida albicans DNA-Fragmente enthält, ein Plasmid, das Candida tropicalis DNA-Fragmente enthält, ein Plasmid, das Candida parapsilosis DNA-Fragmente enthält, ein Plasmid, das Candida krusei DNA-Fragmente enthält, und ein Plasmid, das Candida glabrata DNA-Fragmente enthält; wobei Nukleotidsequenzen der Candida albicans-DNA-Fragmente, der Candida tropicalis-DNA-Fragmente, der Candida parapsilosis-DNA-Fragmente, der Candida krusei-DNA-Fragmente und der Candida glabrata-DNA-Fragmente jeweils als SEQ ID NO. 25, SEQ ID NO. 26, SEQ ID NO. 27, SEQ ID NO. 28 und SEQ ID NO. 29 gezeigt sind; wobei die Hilfsreagenzien ein Taq-Enzym, dNTP, MgC12, DMSO und eine Pufferlösung umfassen;wobei die Pufferlösung Tris-HCl und KC1 umfasst; wobei eine Konzentration der Tris-HCl in der Pufferlösung 15-20 mM beträgt; und wobei eine Konzentration des KC1 in der Pufferlösung 100-200 mM beträgt.

8. Verfahren zum Nachweis getrennter Candida-Typen unter Verwendung des Kits nach einem der Ansprüche 5 bis 7 zum Zwecke der Nicht-Krankheitsdiagnose, umfassend die folgenden Schritte:
(1) Extrahieren von Proben-DNA;
(2) Hinzufügen eines Taq-Enzyms, dNTP, MgC12, DMSO, einer Pufferlösung und der Primer-Sonden-Kombination von fünf Candida-Arten nach einem der Ansprüche 1 bis 6 zur Proben-DNA in Schritt (1); und
(3) Durchführen einer Echtzeit-Fluoreszenz-PCR-Reaktion;
nach dem Schritt (3) umfasst das Verfahren ferner einen Beurteilungsschritt wie folgt:
(a) wenn der Ct-Wert des Nachweiskanals nicht größer als 35 ist, ist das Ergebnis positiv; und wenn der Ct-Wert des Nachweiskanals größer als 35 ist, ist das Ergebnis negativ; und
(b) wobei das positive Ergebnis weiter analysiert wird; wobei aufgrund der Schmelzkurvenanalyse die entsprechenden Glühtemperaturwerte Tm des Produkts und fünf Arten der positiven Qualitätskontrolle gegenübergestellt werden; und wenn die Tm-Werte gleich sind, wird davon ausgegangen, dass die zu detektierenden Proben Candida-Typen enthalten, die der positiven Qualitätskontrolle entsprechen.

9. Verfahren nach Anspruch 8, wobei in Schritt (2) eine Endkonzentration der DNA-Probe 0,1 pg-10 ng beträgt; eine Endkonzentration des Taq-Enzyms 0,5-5 U beträgt; eine Endkonzentration des dNTP 0. 1-5 M beträgt; ein Volumenprozentsatz des DMSO 1-8% beträgt; ein Volumenprozentsatz des DMSO 5% beträgt; eine Endkonzentration spezifischer Primer der fünf Candida-Arten 50-500 nM beträgt; eine Endkonzentration einer Nachweissonde der fünf Candida-Arten 50-500 nM beträgt; und eine Endkonzentration einer komplementären Sonde der fünf Candida-Arten 50-500 nM beträgt.

10. Verfahren nach Anspruch 8, wobei die Bedingungen der Echtzeit-Fluoreszenz-PCR-Reaktion in Schritt (3) wie folgt sind:
(1') Vordegeneration bei 90-98 °C. für 1-10 Minuten und 1 Zyklus;
(2') Degeneration bei 90-98 °C. für 10 s; Verlängerung bei 55-60 °C. für 35-45 s; und 40-50 Zyklen; und
(3') Erhitzung auf 35-97 °C; und 1 Zyklus.

11. Verfahren nach Anspruch 8 oder 9, wobei die Echtzeit-Fluoreszenz-PCR-Reaktion in Schritt (3) insbesondere die folgenden Schritte umfasst:
(1") Vorwärmen auf 50 °C. für 2 Minuten und Durchführen von 1 Zyklus;
(2") Durchführen einer Vordegeneration bei 95 °C für 10 Minuten und Durchführen von 1 Zyklus;
(3") Durchführen der Degeneration bei 95 °C für 10 s; Durchführen der Verlängerung bei 58 °C für 40 s; Erfassen eines Fluoreszenzsignals während der Verlängerung; und Durchführen von 45 Zyklen;
(4") Halten der Temperatur bei 95 °C für 5 s und bei 35 °C für 1 min; Erhöhen der Temperatur auf 97°C mit 0,2 °C pro Sekunde; kontinuierliches Erfassen des Fluoreszenzsignals für 5 mal pro °C.

12. Anwendung der Primer-Sonden-Kombination nach einem der Ansprüche 1 bis 4 und/oder des Kits nach einem der Ansprüche 5 bis 7 zum Nachweis separater Candida-Typen oder zur Herstellung eines Reagens zum Nachweis getrennter Candida-Typen zum Zweck der Nicht-Krankheitsdiagnose.

## Revendications

1. Combinaison d'amorce et de sonde pour détecter des espèces distinctes de *Candida,* comprenant des amorces spécifiques, des sondes de détection et des sondes complémentaires spécifiés à *Candida albicans, Candida tropicalis, Candida parapsilosis, Candida krusei* et *Candida glabrata* respectivement, dans laquelle les sondes complémentaires et les sondes de détection sont appariées de manière complémentaire incomplète ;
dans laquelle des séquences nucléotidiques des amorces spécifiques du *Candida albicans* sont présentées par SEQ ID NO. 1-2 ; des séquences nucléotidiques des amorces spécifiques du *Candida tropicalis* sont présentées par SEQ ID NO.3-4 ; des séquences nucléotidiques des amorces spécifiques du *Candida parapsilosis* sont présentées par SEQ ID NO.5-6 ; des séquences nucléotidiques des amorces spécifiques du *Candida krusei* sont présentées par SEQ ID NO.7-8 ; des séquences nucléotidiques des amorces spécifiques du *Candida glabrata* sont présentées par SEQ ID NO.9-10 ;
une séquence nucléotidique de la sonde de détection du *Candida albicans* est présentée par SEQ ID NO. 11 ; une séquence nucléotidique de la sonde de détection du *Candida tropicalis* est présentée par SEQ ID NO. 12 ; une séquence nucléotidique de la sonde de détection du *Candida parapsilosis* est présentée par SEQ ID NO. 13 ; une séquence nucléotidique de la sonde de détection du *Candida krusei* est présentée par SEQ ID NO. 14 ; une séquence nucléotidique de la sonde de détection du *Candida glabrata* est présentée par SEQ ID NO.15 ;
une séquence nucléotidique de la sonde complémentaire du *Candida albicans* est présentée par SEQ ID NO. 16 ; une séquence nucléotidique de la sonde complémentaire du *Candida tropicalis* est présentée par SEQ ID NO. 17 ; une séquence nucléotidique de la sonde complémentaire du *Candida parapsilosis* est présentée par SEQ ID NO. 18 ; une séquence nucléotidique de la sonde complémentaire du *Candida krusei* est présentée par SEQ ID NO. 19 ; une séquence nucléotidique de la sonde complémentaire du *Candida glabrata* est présentée par SEQ ID NO.20 ;
dans laquelle un extincteur est modifié à l'extrémité 3' de la sonde de détection de chacun des *Candida albicans, Candida tropicalis, Candida parapsilosis, Candida krusei* et *Candida glabrata* ; et un fluorophore est modifié à l'extrémité 5' de la sonde de détection de chacun des *Candida albicans, Candida tropicalis, Candida parapsilosis, Candida krusei* et *Candida glabrata*; et un fluorophore est modifié aux extrémités 5' des sondes de détection sont les mêmes.

2. Combinaison d'amorce et de sonde selon la revendication 1, dans laquelle la combinaison d'amorce et de sonde comprend en outre un système de référence interne ; le système de référence interne comprend des amorces de référence interne et une sonde de référence interne ; des séquences nucléotidiques des amorces de référence interne sont présentées par SEQ ID NO.21-22 ; et une séquence nucléotidique de la sonde de référence interne est présentée par SEQ ID NO.23.

3. Combinaison d'amorce et de sonde selon la revendication 1, dans laquelle un extincteur est modifié à une extrémité 3' de la sonde complémentaire des *Candida albicans, Candida tropicalis, Candida parapsilosis, Candida krusei* et *Candida glabrata.*

4. Combinaison d'amorce et de sonde selon la revendication 1 ou 3, dans laquelle le fluorophore comprend l'un quelconque ou une combinaison d'au moins deux parmi ALEX-350, Alexa Fluor 488, CY3, FAM, VIC, TET, CALGold540, JOE, HEX, CALFluorOrange560, TAMRA, CALFluorRed590, ROX, CALFluorRed610, TexasRed, CALFluorRed635, Quasar670, CY5, CY5.5, LC RED640 ou Quasar705 ;
l'extincteur comprend l'un quelconque ou une combinaison d'au moins deux parmi TAMRA, DABCYL, BHQ-1, BHQ-2, BHQ-3 ou Eclipse.

5. Kit pour détecter des espèces distinctes de *Candida,* comprenant la combinaison d'amorce et de sonde selon l'un quelconque des revendications 1 à 4.

6. Kit pour détecter des espèces distinctes de *Candida* selon la revendication 5, comprenant en outre un contrôle de qualité négatif, un contrôle de qualité positif et des réactifs auxiliaires.

7. Kit pour détecter des espèces distincts de *Candida* selon la revendication 6, dans lequel le contrôle de qualité négatif consiste en des plasmides contenant des fragments d'ADN d'arabidopsis ; une séquence nucléotidique des fragments d'ADN d'arabidopsis est présentée par SEQ ID NO.24 ; le contrôle de qualité positif consiste respectivement en un plasmide contenant des fragments d'ADN de *Candida albicans,* un plasmide contenant des fragments d'ADN de *Candida tropicalis,* un plasmide contenant des fragments d'ADN de *Candida parapsilosis,* un plasmide contenant des fragments d'ADN de *Candida krusei,* un plasmide contenant des fragments d'ADN de *Candida glabrata,* les séquences nucléotidiques des fragments d'ADN de *Candida albicans,* des fragments d'ADN de *Candida tropicalis,* fragments d'ADN de *Candida parapsilosis,* des fragments d'ADN de *Candida krusei,* des fragments d'ADN de *Candida glabrata* sont respectivement présentées par SEQ ID NO.25, SEQ ID NO.26, SEQ ID NO.27, SEQ ID NO.28 et SEQ ID NO.29 ; les réactifs auxiliaires comprennent une enzyme Taq, des dNTP, du MgCl₂, du DMSO et une solution tampon ; la solution tampon comprend du Tris-HCl et du KCl ; la concentration du Tris-HCl dans la solution tampon est de 15-20 mM ; et la concentration du KCl dans la solution tampon est de 100-200 mM.

8. Procédé de détection de espèces distincts de *Candida* en utilisant le kit de l'une quelconque des revendications 5 à 7 dans un but de diagnostic non lié à une maladie, comprenant les étapes suivantes :
(1) l'extraction d'un échantillon d'ADN ;
(2) l'addition d'une enzyme Taq, des dNTP, du MgCl₂, du DMSO, d'une solution tampon et de la combinaison d'amorce et de sonde de cinq espèces de *Candida* selon l'une quelconque des revendications 1 à 6 dans l'échantillon d'ADN de l'étape (1) ; et
(3) la réalisation d'une réaction PCR fluorescente en temps réel ;
après l'étape (3), le procédé comprend en outre une étape de jugement comme suit :
(a) si la valeur Ct du canal de détection est inférieure ou égale à 35, le résultat est positif ; et si la valeur Ct du canal de détection est supérieure à 35, le résultat est négatif ; et
(b) le résultat positif est davantage analysé ; grâce à l'analyse d'une courbe de fusion, des valeurs de température de recuit correspondantes Tm du produit et de cinq espèces de contrôle de qualité positif sont comparées ; et si les valeurs Tm sont les mêmes, on estime que les échantillons à détecter contiennent des espèces de Candida correspondant au contrôle de qualité positif.

9. Procédé selon la revendication 8, dans lequel, à l'étape (2), une concentration finale de l'échantillon d'ADN est de 0,1 pg-10 ng ; une concentration finale de l'enzyme Taq de 0,5 à 5 U ; une concentration finale de dNTP est de 0,1-5 M ; un pourcentage volumique de DMSO est de 1-8 % ; un pourcentage volumique de DMSO est de 5 % ; une concentration finale d'amorces spécifiques des cinq espèces de *Candida* est de 50-500 nM ; une concentration finale d'une sonde de détection des cinq espèces de *Candida* est de 50-500 nM ; et une concentration finale d'une sonde complémentaire des cinq espèces de *Candida* est de 50-500 nM.

10. Procédé selon la revendication 8, dans lequel les conditions de la réaction PCR fluorescente en temps réel à l'étape (3) sont comme suit :
(1') la pré-dégénérescence à 90-98°C pendant 1-10 min, et 1 cycle ;
(2') la dégénérescence à 90-98°C pendant 10 s ; l'extension à 55-60°C pendant 35-45 s ; et 40-50 cycles ; et
(3') le chauffage à 35-97°C ; et 1 cycle.

11. Procédé selon la revendication 8 ou 9, dans lequel la réaction PCR fluorescente en temps réel dans l'étape (3) comprend spécifiquement les étapes suivantes :
(1") le préchauffage à 50°C pendant 2 min, et la réalisation de 1 cycle ;
(2") la réalisation d'une pré-dégénérescence à 95°C pendant 10 min, et la réalisation de 1 cycle ;
(3") la réalisation d'une dégénérescence à 95°C pendant 10 s ; la réalisation d'une extension à 58°C pendant 40 s ; l'acquisition d'un signal de fluorescence pendant l'extension ; et la réalisation de 45 cycles ;
(4") le maintien de la température à 95°C pendant 5 s et à 35°C pendant 1 min ; l'augmentation de la température à 97°C à une vitesse de 0,2°C par seconde ; l'acquisition en continu du signal de fluorescence à 5 fois par °C.

12. Utilisation de la combinaison d'amorce et de sonde de l'une quelconque des revendications 1 à 4 et/ou du kit de l'une quelconque des revendications 5 à 7 pour détecter des espèces distinctes de *Candida* ou pour préparer un réactif pour détecter des espèces distinctes de *Candida* dans un but de diagnostic non lié à une maladie.
